# EUROPÄISCHE PATENTANMELDUNG

(11) **EP 4 091 688 A1**
(43) Veröffentlichungstag der Anmeldung: **23.11.2022**
(21) Anmeldenummer: 21175251.4
(22) Anmeldetag: 21.05.2021
(51) Int. Cl.: B01D 1/28, B01D 3/14, B01D 53/14, C07D 207/26

(54) **ANLAGE UND VERFAHREN ZUM AUFREINIGEN VON RÜCKGEWONNENEM NMP**

(71) Anmelder: GEA Wiegand GmbH, 76275 Ettlingen (DE)
(72) Erfinder: BECKER, Jörg, 76135 Karlsruhe (DE); LEIBIG, Ralf, 76707 Hambrücken (DE)
(74) Vertreter: Herzog, Markus

(57) **Zusammenfassung**

Die vorliegende Erfindung betrifft eine Anlage (10) zum Aufreinigen von rückgewonnenem NMP aus einer Lithium-Ionen-Batterieproduktion, umfassend eine erste Kolonne (16) zum Abtrennen von leichtsiedenden Verunreinigungen, welche in ihrem mittleren Teil eine Zuführung (16a) für das rückgewonnene NMP aufweist und welche mittels eines ersten Verdampfers (18) durch Zufuhr von thermischer Energie beheizbar ist; und eine zweite Kolonne (38) zum Abtrennen von hochsiedenden Verunreinigungen, wobei eine Verbindungsleitung (36) von einem Unterteil (16c) der ersten Kolonne (16) zu einem Unterteil (38c) der zweiten Kolonne (38) vorgesehen ist. Hierbei ist erfindungsgemäß die zweite Kolonne (38) mittels eines zweiten Verdampfers (40) mit einem Eingang (40a) und einem Ausgang (40b) für gereinigtes NMP beheizbar, und der zweiten Kolonne (38) ein Verdichter-Abschnitt (44) zugeordnet, welcher von einem Kopf (38b) der zweiten Kolonne (38) zu dem Eingang (40a) des zweiten Verdampfers (40) verläuft und einen mechanischen Brüdenverdichter (46) zum Eingaben von Energie an den Brüden der zweiten Kolonne (38) umfasst. Weiterhin betrifft die vorliegende Erfindung ein Verfahren zum Betreiben einer derartigen Anlage (10).

## Beschreibung

Die vorliegende Erfindung betrifft eine Anlage zum Aufreinigen von rückgewonnenem NMP aus einer Lithium-Ionen-Batterieproduktion sowie ein Verfahren zum Aufreinigen von rückgewonnenem NMP mittels einer derartigen Anlage.

Insbesondere bei der Herstellung von Elektroden für Lithium-Ionen-Batterien wird als Lösungsmittel NMP (N-Methyl-2-pyrrolidon) in einer besonders hohen Qualität benötigt. Das verwendete NMP wird anschließend in einer Trocknungsanlage aus den Elektrodenmaterialien ausgedampft und nachfolgend kondensiert, um für eine Wiederverwendung rückgewonnen zu werden.

Verfahrensbedingt enthält das rückgewonnene kondensierte NMP, das bei Normalbedingungen einen Siedepunkt von etwa 200°C aufweist, einerseits leicht siedende Verunreinigungen, beispielsweise Wasser, und andererseits hoch siedende Verunreinigungen, beispielsweise Rückstände von Elektrodenmaterial.

Es sind unterschiedliche Verfahren zur Aufreinigung des rückgewonnenen NMP bekannt, wobei beispielsweise auf die JP 2009 212 426 A verwiesen sein soll, in welcher in einem zweistufigen Prozess zunächst einmal leicht siedende Verunreinigungen in einer ersten Kolonne abgetrennt werden, woraufhin in einer zweiten Kolonne das vorgereinigte NMP dann selbst von den hoch siedenden Verunreinigungen abgetrennt wird. In dem genannten Beispiel wird die zur Aufreinigung notwendige Energie in beiden Kolonnen durch thermische Energie, beispielsweise Dampf oder Wärmeträgerflüssigkeit, eingebracht.

Hierbei zeigt es sich jedoch, dass durch diese Art der Energieeingabe ein hoher Energieverbrauch und hohe laufende Kosten beim Betrieb der entsprechenden Anlage entstehen. Es besteht demzufolge Verbesserungspotential hinsichtlich der Energieeffizienz sowie der Betriebskosten von aus dem Stand der Technik bekannten Anlagen und Verfahren zum Aufreinigen von rückgewonnenem NMP aus einer Lithium-Ionen-Batterieproduktion.

Die Möglichkeit, thermische Energie durch den Einsatz von Wärmepumpentechnologie zu reduzieren, wird jedoch zunächst einmal durch die sehr hohe Temperaturdifferenz von über 80 Kelvin zwischen der Kondensationstemperatur des Kopfprodukts (insbesondere der Siedetemperatur von Wasser) und der Verdampfungstemperatur des NMP im Sumpf der Kolonne erschwert.

Hierbei ist andererseits zu beachten, dass verfahrensbedingt das NMP bei seiner Rückgewinnung typischerweise mit weniger als 5 % Wasser- bzw. Leichtsiederanteil anfällt, wodurch während der Aufreinigung des NMP nur ein geringer Energieanteil zur Abreicherung der Leichtsieder aufgewendet werden muss, während der wesentliche Anteil der Energie zum Verdampfen des NMP bzw. zur Schwersiederabtrennung in einem zweiten Prozessschritt benötigt wird.

Demzufolge ist es die Aufgabe der vorliegenden Erfindung, bekannte Anlagen und Verfahren zum Aufreinigen von rückgewonnenem NMP in einer Weise weiterzubilden, die eine erhöhte Energieeffizienz sowie einen kostengünstigeren Betrieb ermöglicht.

Zu diesem Zweck umfasst eine erfindungsgemäße Anlage zum Aufreinigen von rückgewonnenem NMP aus einer Lithium-Ionen-Batterieproduktion eine erste Kolonne zum Abtrennen von leichtsiedenden Verunreinigungen, welche in ihrem mittleren Teil eine Zuführung für das rückgewonnene NMP aufweist und welche mittels eines ersten Verdampfers durch Zufuhr von thermischer Energie beheizbar ist, sowie eine zweite Kolonne zum Abtrennen von hochsiedenden Verunreinigungen, wobei eine Verbindungsleitung von einem Unterteil der ersten Kolonne zu einem Unterteil der zweiten Kolonne vorgesehen ist. Erfindungsgemäß ist hierbei die zweite Kolonne mittels eines zweiten Verdampfers mit einem Eingang und einem Ausgang für gereinigtes NMP durch Kondensation der gereinigten NMP-Brüden beheizbar und der zweiten Kolonne ist ein Verdichter-Abschnitt zugeordnet, weleher von einem Kopf der zweiten Kolonne zu dem Eingang des zweiten Verdampfers verläuft und einen mechanischen Brüdenverdichter zur Druckerhöhung und somit zur Erhöhung der Kondensationstemperatur der gereinigten NMP-Brüden der zweiten Kolonne umfasst.

Demzufolge beruht die Funktionsweise der erfindungsgemäßen Anlage darauf, einen Teil der zur Reinigung des zugeführten NMP notwendigen Energie mittels mechanischer Brüdenverdichtung nach dem Wärmepumpenprinzip einzubringen. Hierbei ist zum Erreichen der erforderlichen Produktreinheit eine Anordnung gewählt worden, bei der die beiden Trennaufgaben, nämlich Leichtsiederabtrennung und Hochsiederabtrennung, auf zwei Kolonnen aufgeteilt sind. Hieraus ergibt sich für die zweite Trennaufgabe, also der Hochsiederabtrennung, eine deutlich reduzierte Temperaturdifferenz zwischen der Kondensationstemperatur des Kopfprodukts, nämlich des aufgereinigten NMP, und der Verdampfungstemperatur des mit Hochsiedern verunreinigten NMP im Sumpf der Kolonne.

Auf diese Weise eignet sich für jene zweite Trennaufgabe insbesondere der Einsatz einer mechanischen Brüdenverdichtung, und mit einer Leistungszahl, definiert als thermische Heizleistung geteilt durch elektrische Leistung der mechanischen Brüdenverdichtung, von etwa 20 im praktischen Betrieb kann bei dieser Trennaufgabe eine deutlich erhöhte Energieeffizienz gegenüber den oben beschriebenen bekannten Verfahren erreicht werden.

Hierbei spielt eine wesentliche Rolle, dass für diese zweite Trennaufgabe das komplette NMP inklusive des erforderlichen Rücklaufs verdampft werden muss und hierfür mehr als 80 % der Gesamtenergie des Betriebs der kompletten erfindungsgemäßen Anlage aufgewendet werden müssen. Somit kann angesichts der oben genannten praktisch erreichbaren Leistungszahl durch den Einsatz der mechanischen Brüdenverdichtung eine Energieeinsparung der Gesamtanlage von bis zu 75 % erzielt werden, wodurch die Installation und der Betrieb des mechanischen Brüdenverdichters bereits in kurzer Zeit amortisiert werden können.

Um die Effizienz der erfindungsgemäßen Anlage weiter zu erhöhen, kann der Zuführung der ersten Kolonne ein Wärmetauscher, insbesondere ein Plattenwärmetauscher, vorgelagert sein, welcher andererseits mit dem Ausgang des zweiten Verdampfers gekoppelt ist. Hierdurch kann das aus der Anlage entnommene rückgewonnene NMP einen weiteren Teil seiner während des Prozesses aufgenommenen Wärme an das zuzuführende rückzugewinnende NMP abgeben, wodurch die entsprechende Energie in der Anlage verbleibt und dementsprechend nicht nachgeliefert werden muss oder verlorengeht.

Weiterhin kann alternativ oder zusätzlich die erfindungsgemäße Anlage ferner einen Kondensator zum Kondensieren der leichtsiedenden Verunreinigung umfassen, welcher mit dem Kopf der ersten Kolonne gekoppelt ist. Das derart erzeugte Kondensat kann einerseits aus der Anlage als Abfallprodukt entnommen werden und andererseits auch teilweise als Rücklauf in die erste Kolonne zurückgeführt werden.

Besondere Eignung für die Verwendung als der erste und/oder der zweite Verdampfer weisen unterschiedliche Bauformen von Fallstromverdampfern oder Umlaufverdampfern auf, während jedoch prinzipiell auch der Einsatz anderer Verdampfertypen an dieser Stelle zur Eingabe der für die entsprechenden Prozesse notwendigen Wärme in die Anlage denkbar ist.

Wie bereits angedeutet, betrifft die vorliegende Erfindung gemäß einem zweiten Aspekt ein Verfahren zum Aufreinigen von rückgewonnenem NMP aus einer Lithium-lonen-Batterieproduktion mittels einer erfindungsgemäßen Anlage der eben beschriebenen Art, umfassend die Schritte:
- Eingeben des rückgewonnenen NMP in den mittleren Teil der ersten Kolonne;
- Beheizen der ersten Kolonne mittels des ersten Verdampfers durch Zufuhr von thermischer Energie;
- Entnehmen von leichtsiedenden Verunreinigungen aus dem Kopf der ersten Kolonne;
- Überführen von vorgereinigtem NMP aus dem Unterteil der ersten Kolonne in das Unterteil der zweiten Kolonne;
- Verdampfen des vorgereinigten NMPs in dem zweiten Verdampfer und Leiten davon durch die zweite Kolonne;
- Entnehmen des dampfförmigen NMP an dem Kopf der zweiten Kolonne und Verdichten davon in dem Verdichter-Abschnitt mittels des mechanischen Brüdenverdichters;
- Eingeben des verdichtenden NMP in den Eingang des zweiten Verdampfers, wobei das NMP in dem zweiten Verdampfer kondensiert;
- Entnehmen des kondensierten NMP an dem Ausgang des zweiten Verdampfers; und
- Entnehmen von hochsiedenden Verunreinigungen aus dem Sumpf der zweiten Kolonne.

Hierbei ist aus den oben angeführten Gründen mit dem erfindungsgemäßen Verfahren eine erhebliche Einsparung an Energie- und Betriebskosten im Betrieb der Anlage zu erzielen.

Weiterhin kann zur Senkung der Betriebstemperatur und damit zur weiteren Energieeinsparung in der ersten und der zweiten Kolonne ein gegenüber Normalbedingungen verminderter Druck aufrechterhalten werden, insbesondere von weniger als 100 mbar Absolutdruck.

Wenngleich die Art und Weise des Zuführens von thermischer Energie zu dem ersten Verdampfer im Rahmen der vorliegenden Erfindung mittels beliebiger Techniken bewerkstelligt werden kann, so kann dies insbesondere mittels Dampf oder einer Wärmeträgerflüssigkeit erfolgen, um die bereits angesprochene hohe Heiztemperatur zum Betrieb der ersten Kolonne erzielen zu können.

Weiterhin kann im Betrieb der erfindungsgemäßen Anlage gemäß dem hier vorgestellten Verfahren ein Teil des kondensierten NMP aus dem zweiten Verdampfer als Rücklauf in die zweite Kolonne zurückgeführt werden.

Wie bereits weiter oben angedeutet, können ferner die dem Kopf der ersten Kolonne entnommenen leichtsiedenden Verunreinigungen in dem Kondensator kondensiert und vorzugsweise teilweise als Rücklauf in die erste Kolonne rückgeführt werden.

Während verfahrensbedingt das rückgewonnene NMP typischerweise weniger als 5 % an leichtsiedenden Verunreinigungen enthalten kann, so können ferner in der ersten Kolonne die leicht siedenden Verunreinigungen in dem vorgereinigten NMP auf weniger als 0,1 %, vorzugsweise auf weniger als 0,05%, abgereichert werden, um die erforderliche Produktqualität für das letztlich aus der Anlage entnommene gereinigte NMP erzielen zu können.

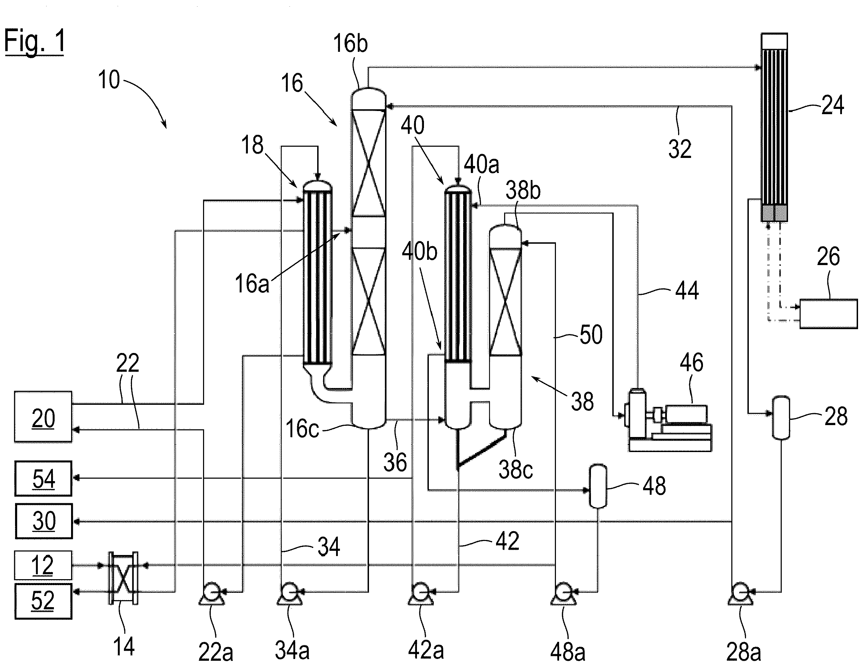

Weitere Merkmale und Vorteile der vorliegenden Erfindung werden aus der nachfolgenden Beschreibung einer Ausführungsform davon noch deutlicher, wenn diese zusammen mit der beiliegenden Figur 1 betrachtet wird. Diese zeigt im Einzelnen:
- Figur 1: eine schematische Darstellung einer erfindungsgemäßen Anlage zum Aufreinigen von rückgewonnenem NMP.

In Figur 1 ist die erfindungsgemäße Anlage zum Aufreinigen von rückgewonnenem NMP ganz allgemein mit dem Bezugszeichen 10 bezeichnet. Hierbei wird zunächst beim Punkt 12 rückgewonnenes NMP aus einer Lithium-Ionen-Batterieproduktion in die Anlage eingegeben, welches typischerweise weniger als 5 % an leichtsiedenden Verunreinigungen, insbesondere Wasser, sowie zusätzlich hochsiedende Verunreinigungen, wie beispielsweise verbleibendes Elektrodenmaterial, enthält.

Das in die Anlage eingegebene NMP durchläuft in der Anlage 10 zunächst einen Wärmetauscher 14, in welchem es vorgewärmt wird, bevor es beim Punkt 16a in den mittleren Teil einer ersten Kolonne 16 eingegeben wird. Hierbei ist der ersten Kolonne 16 ein erster Verdampfer 18 zugeordnet, welchem vom Punkt 20 Dampf oder Wärmeträgerflüssigkeit mittels eines von einer Pumpe 22a angetriebenen Kreislaufs 22 zum Betrieb zugeführt werden kann. Durch die Zuführung von thermischer Energie in die erste Kolonne 16 mittels ersten Verdampfers 18 verdampfen dort die leicht siedenden Verunreinigungen des zugeführten, rückzugewinnenden NMP und können am Kopfbereich 16b der ersten Kolonne entnommen und einem Kondensator 24 zugeführt werden, in welchem mittels einer Wasserkühlung 26 eine Kondensation der leicht siedenden Verunreinigungen erzielt wird. Diese kondensierten Verunreinigungen können anschließend in einem Tank 28 gesammelt und mittels einer Pumpe 28a am Punkt 30 als Abfallprodukt aus der Anlage entnommen sowie teilweise über eine Rückführleitung 32 als Rücklauf in die erste Kolonne 16 zurückgeführt werden.

Demhingegen ist am Unterteil 16c der ersten Kolonne einerseits ein Umwälzkreislauf 34 mit einer Pumpe 34a zum Zuführen von dort entnommenem vorgereinigtem NMP zu dem ersten Verdampfer 18 sowie andererseits eine Verbindungsleitung 36 vorgesehen, über welche das vorgereinigte NMP vom Unterteil 16c der ersten Kolonne 16 in das Unterteil 38c einer zweiten Kolonne 38 überführt werden kann. Die zweite Kolonne 38 ist dazu betreibbar, das bereits vorgereinigte NMP von hoch siedenden Verunreinigungen zu trennen, indem das NMP selbst verdampft und im Kopfbereich 38b dieser zweiten Kolonne 38 entnommen wird.

Hierzu ist dieser zweiten Kolonne 38 ein zweiter Verdampfer 40 zugeordnet, welchem einerseits mittels eines durch eine Pumpe 42a angetriebenen Kreislaufs 42a dem Unterteil 38c der zweiten Kolonne 38 entnommenes, mit hochsiedenden Verunreinigungen angereichertes Sumpfprodukt zugeführt wird, während andererseits zur Beheizung des zweiten Verdampfers 40 der im Kopfbereich 38b der zweiten Kolonne 38 entnommene Brüden, d.h. verdampftes gereinigtes NMP, zugeführt wird. Dieser Brüden wurde zwischenzeitlich in einem von dem Kopfbereich 38b der zweiten Kolonne 38 zu dem entsprechenden Eingang 40a des zweiten Verdampfers 40 verlaufenden Verdichter-Abschnitt 44 mittels eines mechanischen Brüdenverdichters 46 verdichtet und so in seinem Energiegehalt und seiner Kondensationstemperatur erhöht, wodurch in dieser Weise die für den Betrieb der zweiten Kolonne 38 notwendige Energie eingegeben wird.

Bei Durchlauf durch den zweiten Verdampfer 40, welcher beispielsweise als Fallstromverdampfer oder Umlaufverdampfer ausgeführt sein kann, was im Übrigen ebenfalls für den ersten Verdampfer 18 gilt, kondensiert das gereinigte NMP und kann anschließend von einem Ausgang 40b des zweiten Verdampfers 40 in einen Tank 48 überführt werden. Von dort aus kann es anschließend mittels einer Pumpe 48a einerseits über eine Rückführleitung 50 als Rücklauf in die zweite Kolonne 38 zurückgeführt werden und andererseits über eine Ausgangsleitung 52 als Endprodukt aus der Anlage 10 entnommen werden.

Das gereinigte und kondensierte NMP kann abschließend bei einem Durchlauf durch den oben bereits angesprochenen Wärmetauscher 14 weitere Wärme an das am Punkt 12 neu zugeführte, rückzugewinnende NMP abgeben.

In der erfindungsgemäßen Anlage 10 wird erfindungsgemäß durch die Verwendung des mechanischen Brüdenverdichters 46 zur Beheizung des zweiten Verdampfers 40 eine erhebliche Energieeinsparung im Betrieb erzielt, wobei der Vollständigkeit halber zuletzt noch auf die Entnahmeleitung 54 hingewiesen sein soll, mittels welcher die hoch siedenden Verunreinigungen aus dem Unterteil 38c der zweiten Kolonne 38, welche teilweise, wie oben angesprochen, als Rücklauf in den zweiten Verdampfer 40 umgewälzt werden, ebenfalls aus der Anlage 10 als Abfallprodukt entnommen werden können.

## Patentansprüche

1. Anlage (10) zum Aufreinigen von rückgewonnenem NMP aus einer Lithium-Ionen-Batterieproduktion, umfassend:
- eine erste Kolonne (16) zum Abtrennen von leichtsiedenden Verunreinigungen, welche in ihrem mittleren Teil eine Zuführung (16a) für das rückgewonnene NMP aufweist und welche mittels eines ersten Verdampfers (18) durch Zufuhr von thermischer Energie beheizbar ist; und
- eine zweite Kolonne (38) zum Abtrennen von hochsiedenden Verunreinigungen,
wobei eine Verbindungsleitung (36) von einem Unterteil (16c) der ersten Kolonne (16) zu einem Unterteil (38c) der zweiten Kolonne (38) vorgesehen ist;
**dadurch gekennzeichnet, dass** die zweite Kolonne (38) mittels eines zweiten Verdampfers (40) mit einem Eingang (40a) und einem Ausgang (40b) für gereinigtes NMP beheizbar ist, und
der zweiten Kolonne (38) ein Verdichter-Abschnitt (44) zugeordnet ist, welcher von einem Kopf (38b) der zweiten Kolonne (38) zu dem Eingang (40a) des zweiten Verdampfers (40) verläuft und einen mechanischen Brüdenverdichter (46) zum Eingaben von Energie an den Brüden der zweiten Kolonne (38) umfasst.

2. Anlage (10) nach Anspruch 1,
wobei der Zuführung (16a) der ersten Kolonne (16) ein Wärmetauscher (14), insbesondere ein Plattenwärmetauscher, vorgelagert ist, welcher andererseits mit dem Ausgang (40b) des zweiten Verdampfers (40) gekoppelt ist.

3. Anlage (10) nach einem der Ansprüche 1 und 2,
ferner umfassend einen Kondensator (24) zum Kondensieren der leichtsiedenden Verunreinigungen, welcher mit dem Kopf (16b) der ersten Kolonne (16) gekoppelt ist.

4. Anlage (10) nach einem der Ansprüche 1 bis 3,
wobei der erste und/oder der zweite Verdampfer (18, 40) als Fallstromverdampfer oder Umlaufverdampfer ausgeführt ist/sind.

5. Verfahren zum Aufreinigen von rückgewonnenem NMP aus einer Lithium-Ionen-Batterieproduktion mittels einer Anlage (10) nach einem der vorhergehenden Ansprüche, umfassend die Schritte:
- Eingeben des rückgewonnenen NMP in den mittleren Teil der ersten Kolonne (16);
- Beheizen der ersten Kolonne (16) mittels des ersten Verdampfers (18) durch Zufuhr von thermischer Energie;
- Entnehmen von leichtsiedenden Verunreinigungen an dem Kopf (16b) der ersten Kolonne (16);
- Überführen von vorgereinigtem NMP aus dem Unterteil (16c) der ersten Kolonne (16) in das Unterteil (38c) der zweiten Kolonne (38);
- Verdampfen des vorgereinigten NMP in dem zweiten Verdampfer (40) und Leiten davon durch die zweite Kolonne (38);
- Entnehmen des dampfförmigen NMP an dem Kopf (38b) der zweiten Kolonne (38) und Verdichten davon in dem Verdichter-Abschnitt (44) mittels des mechanischen Brüdenverdichters (46);
- Eingeben des verdichteten NMP in den Eingang (40a) des zweiten Verdampfers (40), wobei das NMP in dem zweiten Verdampfer (40) kondensiert;
- Entnehmen des kondensierten NMP an dem Ausgang (40b) des zweiten Verdampfers (40); und
- Entnehmen von hochsiedenden Verunreinigungen aus dem Sumpf (38c) der zweiten Kolonne (38).

6. Verfahren nach Anspruch 5,
wobei in der ersten und der zweiten Kolonne (16, 38) ein verminderter Druck aufrecht erhalten wird, insbesondere von weniger als 100 mbar.

7. Verfahren nach einem der Ansprüche 5 und 6,
wobei das Zuführen von thermischer Energie zu dem ersten Verdampfer (18) mittels Dampf oder einer Wärmeträgerflüssigkeit erfolgt.

8. Verfahren nach einem der Ansprüche 5 bis 7,
wobei ein Teil des kondensierten NMP aus dem zweiten Verdampfer (40) als Rücklauf in die zweite Kolonne (38) rückgeführt wird.

9. Verfahren nach einem der Ansprüche 5 bis 8,
wobei die an dem Kopf (16b) der ersten Kolonne (16) entnommenen leichtsiedenden Verunreinigungen in dem Kondensator (24) kondensiert und vorzugsweise teilweise als Rücklauf in die erste Kolonne (16) zurückgeführt werden.

10. Verfahren nach einem der Ansprüche 5 bis 9,
wobei das rückgewonnene NMP weniger als 5 % an leichtsiedenden Verunreinigungen enthält; und/oder
wobei in der ersten Kolonne (16) die leichtsiedenden Verunreinigungen in dem vorgereinigten NMP auf weniger als 0,1%, vorzugsweise aus weniger als 0,05%, abgereichert werden.
